# EUROPEAN PATENT SPECIFICATION

(11) **EP 2 366 474 B1**
(45) Date of publication and mention of the grant of the patent: **22.05.2013**
(21) Application number: 04762187.5
(22) Date of filing: 16.09.2004
(51) Int. Cl.: B22D 2/00, B22D 11/18, G01N 27/90

(54) **DEVICE FOR DETECTING SLAG CONTENT IN LIQUID METAL STREAM**
VORRICHTUNG ZUR ERFASSUNG DES SCHLACKENGEHALTS IN EINEM FLÜSSIGMETALLSTROM
DISPOSITIF DE DETECTION DE LAITIER DANS UN COURANT METALLIQUE LIQUIDE

(30) Priority: 17.09.2003 CN 03156980
(43) Date of publication of application: 21.09.2011
(73) Proprietor: Jiang, Hong, Hengyang, Hunan 421000 (CN)
(72) Inventor: Jiang, Hong, Hengyang, Hunan 421000 (CN)
(74) Representative: Kirschner, Klaus Dieter
(86) International application number: PCT/CN2004/001053
(87) International publication number: WO 2005/035168

(56) References cited:
- CN-A- 1 032 504
- CN-A- 1 349 095
- DE-A1- 3 142 681
- FR-A1- 2 532 208
- GB-A- 1 585 496
- US-A- 4 602 768
- US-A- 4 810 988
- US-A- 4 816 758

## Description

### Field of Technology

The invention relates to technique for detecting slag content in the stream of liquid metal, specifically a device for detecting slag content in the stream of liquid metal through the outlet of a smelting furnace or ladle during the continuous casting process.

### Background Technology

During continuous casting process, there will be some slags flowing out along with liquid metal at the end of the stream of liquid metal through the outlet of the smelting furnace or the ladle. In order to keep the purity of the liquid metal, it is necessary to provide a sensor at the outlet of the smelting furnace or the ladle to detect and control the slag content. The sensor is the key element of the device for detecting slag content in the stream of liquid metal. Usually, it is an annular electromagnetic sensor that surrounds the liquid metal stream. Since the sensor is located at high temperature environment, insulation between coils is a key problem to be solved during manufacturing. In the patent DE 3722795 of AMEPA, a ceramic material filler is used to get an insulation between the coils in such a way that the coils, the ceramic material and a stainless steel sleeve constitute a shield cable. The advantage of this patent involves in simple manufacturing process. The disadvantages reside in the following: firstly, the shield cable is thick, and the number of circles to be wound within the limited space in the sensor is limited (about 10 circles), which causes the inductive impedance resulting in the capability of electromagnetic induction of the coil is lower(less than 0.3 ohm), so the sensitivity of the sensor is limited; secondly, since the inner space in the shield cable is narrow, the core wires therein are thin, its direct current resistance (sending coil is about 50 ohms and receiving coil is about 150 ohms) exceeds much the inductive impedance of the coil, therefore, most part of the exciting power is consumed by the sending coil sending out heat, but only the power portion less than 0.6% is used for electromagnetic excitation, this further limits the sensitivity of the sensor. For this lower sensitivity, the signal amplitude of the sensor should be amplified largely, and hence the stability of the system is worse. In the patent ZL 01255085.X of Zhejiang University, for solving the insulation problem in the high temperature environment, an insulating cetamic tube is fit over the coils around which the insulating material is filled in. Nevertheless, the manufacture process in this solution is complicated on the one hand, and on the other hand in this solution the coil has to be implemented as a single circle structure because the insulating tube and material occupies most part of the space within the sensor, so the sensitivity and stability of the sensor is limited much; up till now, this solution has not been put into application. Another key problem to be solved is how to reduce the background interference signal in the sensor (the change of background signal emanated in the receiving coil by the sending coil and the signal drift caused by the temperature change of the base sheet of the smelting furnace or vessel). In patent DE 3722795,the signal drift in the sensor is reduced by providing locally an electromagnetic shield and providing several currents of different frequencies to the sending coil. For doing so, the sending coil and receiving coil are arranged in the same small chamber in the sensor box (in fact the sending coil and receiving coil are arranged in a shield cable in the same one stainless steel sleeve), and the reference coil is arranged in another chamber in the sensor box or outside the sensor box or some electric signal are used to simulate a reference coil in order that an electromagnetic shield material can be is additionally integrated into different parts inside the sensor box. This solution makes distance between the receiving coil and sending coil shorter, so the mutual inductance coefficient and its temperature coefficient are increased so as to increase the background signal of the receiving coil and a variance of the background signal as a function of temperature, the amount of signal drift produced by temperature change of the sensor itself exceeds that cause by the material temperature change in the base sheet. This affects the sensor stability directly By incoporating a electromagnetic shield material, especially moving the reference coil outside the sensor, the reference coil and the receiving coil are located at different environments; in this case it is difficult to counteract the interference signal in the receiving coil by the reference coil and current excited by additional frequency.

EP 0 300 150 discloses a device for detecting slag flowing with a stream of molten metal discharged through an outlet opening in a metallurgical vessel includes a sensor positioned in the area of a base plate of the vessel to surround the stream of molten metal without being contacted thereby. The sensor has a sending coil and a receiving coil associated with a reference coil. At least the sending and receiving coils are positioned within a housing formed of a non-magnetic material. The housing encompasses the coils and defines there around a protective casing that alters electromagnetic fields emanating from the base plate and the stream of molten metal and that is resistant to mechanical stresses.

Objective of the invention is to provide a sensible, stable and reliable device for detecting slag content in the stream of liquid metal, which can be manufactured easily.

For this purpose, the device for detecting slag contained in a liquid according to the invention comprises the features of claim 1. Preferred embodiments of the invention are characterized in the sub-claims.

The objective of the invention is implemented as follows: producing a device for detecting slag content in liquid metal stream; said device includes a sensor surrounding said stream, a signal cable and a signal processor. Said sensor has an annular metal housing, some layers of coils within the sensor each are wound in a spiral groove that is on an insulation layer [0005] The insulation layer consists of insulation sheets with groove. The insulation sheets with groove as being annularly arranged, displace one by one in the axial direction of the annular sensor in such a way that each sheets displace a circle pitch in the axial direction of the annular sensor when being arranged in a complete circumference. Therefore, the groove in an insulation sheet is a spiral groove that can be used to wind the coil.

In the sensor of the invention, from the inner ring to the outer ring are provided the receiving coil, filling layer, sending coil and compensating coil are positioned sequentially and respectively. Signals in the compensating coil and receiving coil are superposed in opposite phase in the signal processor.

In the sensor of the invention, the insulation sheet with groove in the sensor for detecting slag can accomplish an insulation between every layer of coils and an insulation between every circle in the same layer, and in this way the manufacturing process is simplified, cost of the material is decreased and the manufacturing period is shorten. It is particularly important that the coils wound along the grooves can be wound by plural circles and have a large diameter, so an inductive impedance of the sending coil is increased while a direct current resistance of the sending coil is decreased. Therefore, finally the exciting current in the sending coil is enlarged, especially the emitting electromagnetic energy portion is increased so as to improve the sensor sensitivity.

In the invention, the filling layer incorporated into the sensor for detecting slag causes the coils in the sensor arranged unevenly, so that the sending coil is placed away form the receiving coil and near the compensating coil. In this way, the background signal emanated in the receiving coil by the sending coil is lowered, it can be counteracted by a small number of circles of the compensating coil. In addition, the incorporation of the filling layer increases the distance between the compensating coil and the stream of liquid metal so that the induced signal, emanated by the eddy current, in the compensating coil which is equpped with small number of circls and is located away from it, is much smaller than the induced signal by the eddy current in the receiving coil which is equipped with large number of circles and is located away from it. When the induced signal in the compensating coil counteracting the background signal produced in the receiving coil by the sending coil, the useful signal produced in the receiving coil by the eddy current in liquid metal stream is reduced little. Furthermore, the electromagnetic structural material such as the base sheet, used as emitting body of secondary electromagnetic waveis near the compensating coil with small amount of circles and is away from the receiving coil with large amount of circles, it is useful to suppre the sensor signal drift caused by the electromagnetic material. As a result of that, the filling layer effectively improves the signal-to-noise ratio of the annular sensor for detecting slag stream.

In the invention,the differential structure of the receiving coil and the compensating coil, the surprising signal-to-noise ratio and large exciting current of the sensor in accordance with the present invention ensure that the detecting means for detecting slag content in the liquid metal of the invention is of high sensitivity, stability and reliability.

### Brief Description of Drawings

The present invention is described by the aid of Figurs 1 and 2A-2D.
Figure 1 is the system diagram of the device for detecting slag;
Figure 2A is a front elevation of the sensor for detecting slag;
Figure 2B is a top view of the sensor for detecting slag;
Figure 2C is an enlarged view through line A-A of Figure 2B; and
Figure 2D is an enlarged partial view of Figure 2B.

### Embodiments

The invention will be described in more detail with reference to drawings and embodiments.

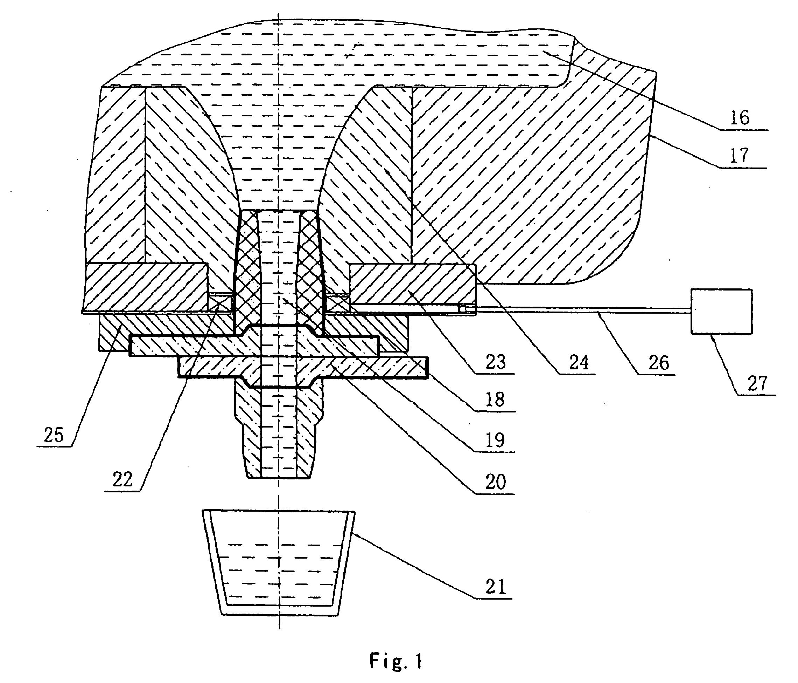

Figure 1 shows a systematic diagram of the device for detecting slag content in the liquid metal stream discharged from a ladle. In Figure 1, the liquid metal 16 is in a ladle 17 at the bottom of which is provided a nozzle 18, and the stream of liquid metal 19 flows into a tundish through the nozzle 18 via an aperture of a slider 20. A sensor of electromagnetic type 22 for detecting slag is provided around the stream of liquid metal 19 and the nozzle 18; the base sheet of the vessel 23 adjoins the outer annular side of the sensor 22, above the sensor is provided adown seating brick 24 of the ladle and underneath the sensor is provided an mounting support of the upper slider 25. The signal of the sensor 22 is transferred to the signal processor 27 through the cable 26. When slag content in the stream of liquid metal 19 exceeds a preset value, the signal processor 27 alarms and sending a signal for closing the closing slider 20 so as to prevent the stream of liquid metal containing excessive slag from flowing into the tundish 21.

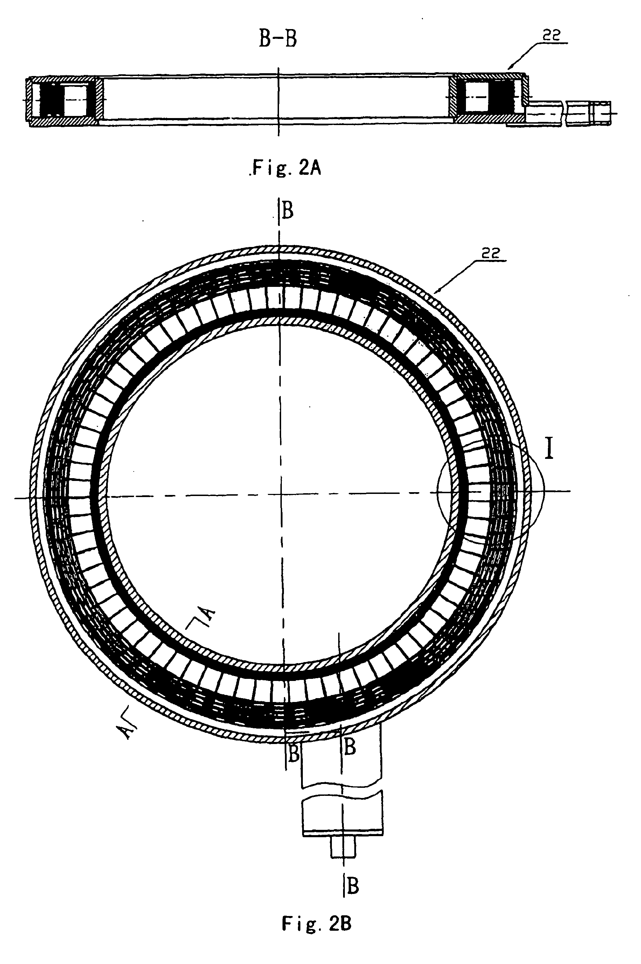

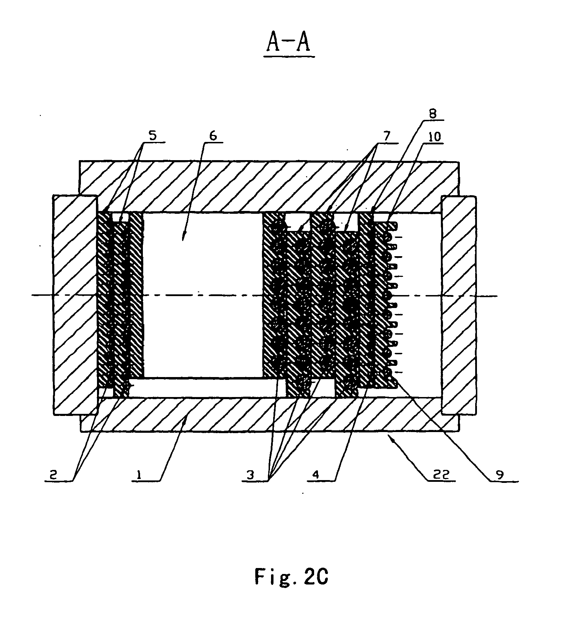

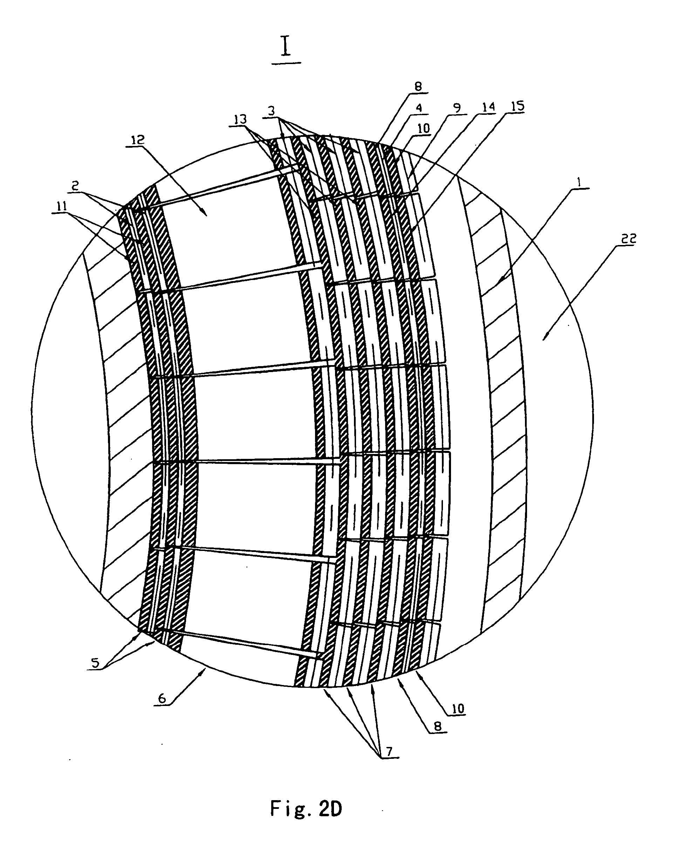

Figures 2A to 2D show the principle and structure of the sensor for detecting slag 22. The sensor 22 is an annular shape with a metal housing 1. Inside the housing 1, from the inner ring to outer ring are provided a receiving coil 2, a sending coil 3 and a compensating coil 4 sequentially and respectively. Two layers of receiving coil 2 are wound in the spiral grooves of two insulation layers with groove. Between the receiving coil 2 and the sending coil 3 is located a filling insulation layer 6 with grooves. Four layers of sending coil 3 are wound in the spiral grooves of the filling insulation layer 6 and three insulation layers 7, respectively. One layer of compensating coil 4 of figures is wound in the spiral groove of one insulation layer 8. Outside the compensating coil 4 is provided one layer of coil used as a fastening layer 9 that is wound in the spiral groove of the insulation layer 10 so as to keep the shape and position of the receiving coil 2, sending coil 3, compensating coil 4, insulation layer 5, filling layer 6, insulation layer 7 and 8 within the sensor 22 constant. The insulation layers 5, 7, 8 and 10 consist of some insulation sheets with grooves 11, 13, 14 and 15, respectively, and the filling insulation layer consists of the filling insulation block with groove 12. When being arranged vertically and by layers in form of annular shape, within the sensor 22 horizontally located, The insulation sheet with grooves 11, 13, 14, 15 and the filling insulation block 12 with grooves each displace along the axial direction of the annular sensor evenly one by one, so that the insulation sheet 11,13,14 and 15 and filling insulation block 12 displace by a circle pitch accumulatively in the axial derection of the annular sensor 22, when they are arranged in a complete circumference to form insulation layers 5,7,8,10 or filling layer 6. In this way, the grooves on the insulation layer 5, 7, 8, 10 and filling layer6 form a spiral groove on which coils can be wound. Usually, the perimeter of the inner coil of the sensor 22 exceeds greatly the width of the insulation sheet 11,13,14,15 and the filling block 12, and the curvature radius of the groove on the insulation sheet 11,13,14,15 and filling block 12 is slightly larger than the radius of wires wound therein so as to ensure that the coil can be wound in the sprial groove smoothly. The insulation sheets 11, 13, 14, 15 and the filling insulation block 12 each are made of ceramic material with high-temperature resistance.

The alternating current generated by the signal processor 27 is fed into the sending coil 3 through the cable 26 so as to induce eddy current in the stream of liquid metal 19, and the eddy current induces signals in the receiving coil 2 and the compensating coil 4, respectively. These two induced signals are transferred to the signal processor 27 through the cable 26 to be superposed in opposite phase therein, so that the background signals in the receiving coil 2 and compensating coil 4 by the sending coil 3 are mutually counteracted. The incorporation of the filling layer 6 makes the distance between these two coils increased, and reduces the background signal in the receiving coil 3 produced by the sending coil 2. Therefore, the compensating coil 4 with a small number of circles can counteract the background signal in the receiving coil 2 with large number of circles produced by the sending coil 3. Compared to the compensating coil 4, the receiving coil 2 has large number of circles and is located next to the stream of liquid metal 19. The eddy current in the liquid metal stream 19 emanated by the sending coil 3, induces a signal in the receiving coil 2 exceeding greatly the signal produced by the eddy current in the compensating coil 4, which has small number of circles and is located away from it. Therefore, when superposing these two signals in opposite phase in the signal processor 27, most part of the signal in the receiving coil 2 produced by the eddy current in the liquid metal stream 19 remains. In addition to the background signal in the receiving coil 2 produced by the sending coil 3, the eddy current produced by the current of the sending coil 3 in the electromagnetic medium materials such as the base sheet 23 of the ladle and the mounting support of the upper slider 25 and the like, is a key factor causing signal in the sensor 22 drifted. In Figure 1, the metal materials such as the base sheet 23 and the installing support of the upper slider 25 and the like are located next to the compensating coil 4 with small number of circles and are located away from the receiving coil 2 with large number of circles; this arrangement advantageously reduces the interference to the sensor caused by temperature change in the electromagnetic material surrounding the sensor 22.

## Claims

1. A device for detecting slag content in liquid metal stream (19) comprising:
a sensor (22) mounted about said stream (19),
a signal cable (26) and a signal processor (27), said sensor (22) comprising a metal housing (1) and a receiving coil (2), a sending coil (3) and compensating coil (4) provided from an inner ring to an outer ring sequentially;
**characterized in that**
said coils (2, 3, 4) are wound in a spiral groove, wherein
said spiral groove supporting said coils (2,3.4) is formed in insulation sheets (5, 7, 8, 10) with
grooves (11, 13, 14, 15), or
said spiral groove supporting said coils (2, 3, 4) is formed in insulation sheets (5, 7, 8, 10) with grooves (11, 13, 14, 15) and an insulation filling block (12) with groove.

2. The device according to Claim 1, **characterized in that** said spiral groove supporting said coils (2, 3, 4)
is formed in such a way that several insulation sheets (5, 7, 8, 10) with grooves (11, 13, 14, 15) are displaced one
by one along the axial direction of said sensor (22) or in such a way that several insulation sheets (5, 7, 8, 10) with grooves (11, 13, 14, 15) and an insulation filling block (12) with grooves are displaced one by one along the axial direction of said sensor (22)_{.}

3. The device according to claim 1, **characterized in that** a filling layer (6) is provided between said receiving coil (2) and the sending coil (3).

4. The device according to Claim 3, **characterized in that** said filling layer (6) of the sensor (22) is a filling insulation layer with spiral groove consisting of an insulation filling block with grooves.

5. The device according to Claim 1, **characterized in that** a fastening coil (9) wound in spiral groove on an insulation layer (10) formed of an insulation sheet with groove is provided outside said compensating coil (4).

## Patentansprüche

1. Vorrichtung zur Erfassung des Schlackengehalts in einem Flüssigmetallstrom (19) umfassend:
einen Sensor (22), der um den Strom (19) herum montiert ist,
ein Signalkabel (26) und ein Signalprozessor (27), wobei der Sensor (22) ein Metallgehäuse umfasst, und eine Empfangsspule (2), eine Sendespule (3) und eine Kompensationsspule (4),
die nacheinander von einem inneren Ring zu einem äußeren Ring hin angeordnet sind;
**dadurch gekennzeichnet, dass**
die Spulen (2, 3, 4) in einer spiralförmigen Nut gewickelt sind, wobei die spiralförmige Nut, die die Spulen (2, 3, 4) trägt, in Isolationsblättern (5, 7, 8, 10) mit Nuten (11, 13, 14, 15) ausgebildet ist, oder
wobei die spiralförmige Nut, die die Spulen (2, 3, 4) trägt, in Isolationsblättern (5, 7, 8, 10) mit Nuten (11, 13, 14, 15) und in einem Isolations-Füllblock (12) mit Nut ausgebildet sind.

2. Vorrichtung nach Anspruch 1, **dadurch gekennzeichnet, dass** die spiralförmige Nut, die die Spulen (2, 3, 4) trägt, in einer solchen Weise ausgeformt ist, dass verschiedene Isolationsblätter (5, 7, 8, 10) mit Nuten (11, 13, 14, 15) einzeln in der axialen Richtung des Sensors (22) versetzt sind oder in einer solchen Weise angeordnet sind, dass mehrere Isolationsblätter (5, 7, 8, 10) mit Nuten (11, 13, 14, 15) und ein Isolations-Füllblock (12) mit Nuten einzeln in der axialen Richtung des Sensors (22) versetzt angeordnet sind.

3. Vorrichtung nach Anspruch 1, **dadurch gekennzeichnet, dass** eine Füllschicht (6) zwischen der Empfangsspule (2) und der Sendespule (3) vorgesehen ist.

4. Vorrichtung nach Anspruch 3, **dadurch gekennzeichnet, dass** die Füllschicht (6) des Sensors (22) eine Isolationsfüllschicht mit spiralförmiger Nut ist, die aus einem Isolationsfüllblock mit Nuten besteht.

5. Vorrichtung nach Anspruch 1, **dadurch gekennzeichnet, dass** eine Befestigungsspule (9) in einer spiralförmigen Nut auf einer Isolierschicht (10) gewickelt ist, die auf einer Isolierschicht mit Nut ausgebildet ist, die außerhalb der Kompensationsspule (4) vorgesehen ist.

## Revendications

1. Dispositif de détection der laitier dans un courant métallique liquide (19) comprenant:
- un capteur (22) monté sur ledit courant (19),
- un câble de signal (26) et un processeur de signaux (27), ledit capteur (22) comprenant un boîtier métallique (1) et une bobine de réception (2), une bobine d'envoi (3) et la bobine de compensation (4) pourvue séquentiellement à partir d'un anneau intérieur vers un anneau extérieur; **caractérisé en ce que**
- lesdites bobines (2, 3, 4) sont enroulées dans une cannelure spirale, où
- ladite cannelure spirale soutenant lesdites bobines (2, 3, 4) est formée dans les feuilles d'isolation (5, 7, 8, 10) avec les cannelures (11, 13, 14, 15), ou
- ladite cannelure spirale soutenant lesdites bobines (2, 3, 4) est formée dans les feuilles d'isolation (5, 7, 8, 10) avec les cannelures (11, 13, 14, 15) et un bloque de remplissage d'isolation (12) à cannelure.

2. Dispositif selon la revendication 1, **caractérisé en ce que** ladite cannelure spirale soutenant lesdites bobines (2, 3, 4) est formée de telle manière que les diverses feuilles d'isolation (5, 7, 8, 10) avec les cannelures 11, 13, 14, 15) sont déplacées l'une après l'autre le long de la direction axiale dudit capteur (22) ou de telle manière que les diverses feuilles d'isolation (5, 7, 8, 10) avec les cannelures (11, 13, 14, 15) et un bloque de remplissage d'isolation (12) à cannelures sont déplacées l'une après l'autre le long de la direction axiale dudit capteur (22).

3. Dispositif selon la revendication 1, **caractérisé en ce qu'**une couche de remplissage (6) est pourvue entre ladite bobine de réception (2) et la bobine d'envoi (3).

4. Dispositif selon la revendication 3, **caractérisé en ce que** ladite couche de remplissage (6) du capteur (22) est une couche d'isolation de remplissage avec la cannelure spirale formée d'un bloque de remplissage d'isolation (12) à cannelures.

5. Dispositif selon la revendication 1, **caractérisé en ce qu'**une bobine de fixation (9) enroulée dans la cannelure spirale sur une couche d'isolation (10) formée d'une feuille d'isolation à cannelure est pourvue à l'extérieur de ladite bobine de compensation (4).
